# EUROPEAN PATENT APPLICATION

(11) **EP 0 924 296 A2**
(43) Date of publication of application: **23.06.1999**
(21) Application number: 98204089.1
(22) Date of filing: 03.12.1998
(51) Int. Cl.: C12N 15/12, C07K 14/47, A61K 38/17, C12Q 1/68, C12N 1/19, C12N 1/21

(54) **Methods and means for inducing apoptosis by interference in RNA processing**

(30) Priority: 03.12.1997 EP 97203782
(71) Applicant: Leadd B.V., 2333 AL Leiden (NL)
(72) Inventor: Noteborn, Mathieu Hubertus Maria, 2352 EH Leiderdorp (NL); Danen-van Oorschot, Astrid Adriana Anna Maria, 2651 VG Berkel en Rodenrijs (NL)
(74) Representative: Ottevangers, Sietse Ulbe

(57) **Abstract**

The invention relates to activation of apoptosis by means of interference with the function of snRNPs and hnRNP-like compounds.

Also the invention relates to anti-tumor therapies with compounds, which negatively interfere with snRNPs and hnRNP-like compounds leading to induction of apoptosis, resulting in the elimination of tumor cells.

Also the invention relates to therapies for diseases related to aberrant apoptosis induction, such as auto-immune diseases.

## Description

The present invention relates to the field of apoptosis, as well as to the field of cancer diagnosis and treatment and diagnosis and treatment of auto-immune diseases and other diseases. In particular the invention relates to improved methods and means for inducing apoptosis in cells to be eliminated. In particular the invention relates to novel means and methods for inducing apoptosis by interfering with the RNA processing machinery of a cell. In particular it relates to inhibiting or modifying the function of RNA-protein complexes involved in RNA processing such as snRNP's and hnRNP's. Both complexes are shown herein to be components of the apoptotic pathway that can be induced by chicken anemia virus proteins VP2 and/or VP3 (also called apoptin), both the hnRNP-like and snRNPs compounds are shown to associate to CAV-derived proteins Apoptin and VP2, which both are known to be involved in the apoptotic process. Apoptin and VP2 as stated, are proteins originally found in chicken anemia virus (CAV; Noteborn et al., 1991; apoptin was originally called VP3. The apoptotic activity of these proteins was discovered by the group of the present inventors (Noteborn et al., 1994, 1997).

Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis, e.g. in cancer development, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Transforming genes of tumorigenic DNA viruses inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).

For such cancers (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by Bcl-2/Bcr-abl-like anti-apoptotic activities. These factors might be part of a distinct apoptosis pathway or being (far) downstream to the apoptosis inhibiting compounds.

Apoptin is a small protein derived from chicken anemia virus (CAV; Noteborn and De Boer, 1995, Noteborn et al., 1991, Noteborn et al., 1994), which can induce apoptosis in human malignant and transformed cell lines, but not in untransformed human cell lines. In vitro, apoptin fails to induce programmed cell death in normal lymphoid, dermal, epidermal, endothelial and smooth-muscle cells. However, when normal cells are transformed they become susceptible to apoptosis by apoptin. (Danen-van Ooschot, 1997 and Noteborn, 1996). Long-term expression of apoptin in normal human; fibroblasts revealed that apoptin has no toxic or transforming activity in these cells.

In normal cells, apoptin was found predominantly in the cytoplasm, whereas in transformed or malignant cells i.e. characterized by hyperplasia, metaplasia or dysplasia, it was located in the nucleus, suggesting that the localization of apoptin is related to its activity (Danen-van Oorschot et al. 1997).

Apoptin-induced apoptosis occurs in the absence of functional p53 (Zhuang et al., 1995a), and cannot be blocked by Bcl-2, Bcr-abl (Zhuang et al., 1995), the Bcl-2-associating protein BAG-1 and not by the caspase-inhibitor cowpox protein CrmA (Danen-Van Oorschot, 1997a, Noteborn, 1996).

Therefore, apoptin is a potent agent for the destruction of tumor cells, or other hyperplasia, metaplasia or dysplasia which have become resistant to (chemo)therapeutic induction of apoptosis, due to the lack of functional p53 and (over)-expression of Bcl-2 and other apoptosis-inhibiting agents (Noteborn et al., 1997).

The fact that apoptin does not induce apoptosis in normal transformed human cells, at least not in vitro, suggests that a toxic effect of apoptin treatment in vivo will be very low. Noteborn et al. (1997) have provided evidence that adenovirus expressed apoptin does not have an acute toxic effect in vivo. In addition, in nude mice it was shown that apoptin has a strong anti-tumor activity.

It appears, that even pre-malignant, minimally transformed cells, may be sensitive to the death-inducing effect of apoptin. In addition, Noteborn and Zhang (1997) have shown that apoptin-induced apoptosis can be used as diagnosis of cancer-prone cells and treatment of cancer-prone cells.

Knowing that apoptin is quite safe in normal cells, but that as soon as a cell becomes transformed and/or immortalized (the terms may be used interchangeable herein) the present inventors designed novel menans and methoids for induction of apoptosis based on the identification of compounds involved in the apoptin-induced apoptotic cascade. These compounds are factors of an apoptosis pathway, which is specific for transformed cells. Therefore, these proteins are very important compounds in new treatments and diagnosis for diseases related with aberrancies in the apoptotic process, such as cancer, and (auto-)immune diseases.
A group of proteins found to be associated with apoptin is the family of hnRNP-like proteins.

The invention provides an apoptin-associating hnRNP-like protein, which is needed for RNA processing. When apoptin associates with such proteins it interferes with normal RNA processing, thus leading to apoptosis.

The invention thus further provides a method for inducing apoptosis through interference with hnRNP-like proteins (interchangeably referred to as hnRNP or hnRNP-like proteins) or other parts of hnRNP's.

The invention provides an anti-tumor therapy based on the interference with hnRNP-like proteins or other parts of hnRNP's.
As an additional mechanism hnRNP can shuttle apoptin or apoptin-like compounds to the nucleus where these compounds can induce apoptosis.
The invention thus provides hnRNP as mediator of apoptin-induced apoptosis, which is tumor-specific.

The present inventors have also shown a colocalization of VP2 with snRNP's another compound also involved in RNA processing.

The invention provides a VP2-associating snRNP-like protein or component, which is needed for RNA processing.

The invention further provides a method for inducing apoptosis through interference with snRNP-like proteins or components (interchangeably referred to as snRNP or snRNP-like proteins).

The invention provides an anti-tumor therapy based on the interference with snRNP-like proteins.

The invention provides snRNP as mediator of VP2-induced apoptosis.

The invention further provides a method for inducing apoptosis through interference with hnRNP-like and snRNP-like proteins.

The invention provides an anti-tumor therapy based on the interference with either or both snRNP- and hnRNP-like proteins.

The invention provides hnRNP and snRNP as mediators of VP2-induced apoptosis.

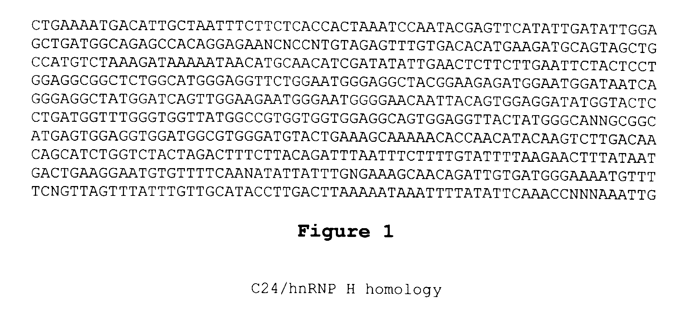

More in detail the invention provides a recombinant and/or isolated nucleic acid molecule encoding a member of the family of hnRNP proteins involved in RNA processing comprising at least a functional part of the sequence of figure 1 or a sequence having at least 60, preferably 70, preferably 80, more preferably 90% homology with said sequence. In cells where a particular hnRNP is not used for RNA processing such hnRNP activity can be used to shuttle apoptotic agents such as apoptin to the nucleus. It is then preferred to have such activity in an expression vector. hnRNP (-like) activity is defined as any molecule directly or indirectly providing the same kind of activity as an hnRNP or an hnRNP-like protein.

Such a vector preferably also encodes apoptotic activity, preferably apoptin-like acctivity which is defined analogous to hnRNP-like activity.
In this definition functional equivalents and/or fragments of apoptin are also encompassed.

In the case where hnRNP's are involved in RNA processing these compounds can be inhibited by apoptin-like activity, but also by for instance antisense molecules for hnRNP components. The invention thus also provides a recombinant and/or isolated nucleic acid molecule encoding an antisense recombinant molecule which can hybridize with a recombinant acid molecule according to claim 1. Preferably again such a molecule is present in an expression vector.

Apoptosis is preferably induced in a gene therapy setting, so that it is preferred to deliver all vectors to cells making use of gene delivery vehicle. Gene delivery vehicle are known in the art and our capable of transporting nucleic acid molecules of interest to cells. They include recombinatn viruses (such as adenoviruses and retroviruses) as well as polymers and liposomes and the like.
It is preferred to also block the snRNP involvement in RNA processing. This can be done by VP2 (or VP2-like activity (same defintion as hnRNP-like activity)) or by a further antisense molecule hybridizing with a nucleic acid molecule encoding a snRNP component.
Both options are provided by the present invention. The invention thus provides an expression vector encoding an antisense molecule for a nucleic acid encoding a component of an snRNP, preferably together with an hnRNP antisense molecule.

The invention also provides a method for identifying apoptotic agents comprising the use of nucleic acid molecules encoding members of the hnRNP-like family and the snRNP-like family.
Apoptotic agents identified by such a route are also considered part of this invention. These agents will typically be hnRNP antagonists or snRNP antagonists of which apoptin and VP2 are the first examples.
The most preferred method of inducing apoptosis is using antagonists to both snRNP and hnRNP, but often singel anatagonists will suffice.

The invention will be explained in more detail in the following experimental part. This only serves for the purpose of illustration and should not be interpreted as a limitation of the scope of the invention.

### EXPERIMENTAL PART

The inventors have used the yeast-2 hybrid system (Durfee et al., 1993) to identify apoptin-associating cellular compounds, that are essential in the induction of apoptosis. The used system is an in-vivo strategy to identify human proteins capable of physically associating with apoptin. It has been used to screen cDNA libraries for clones encoding proteins capable of binding to a protein of interest (Fields and Song, 1989, Yang et al., 1992).

### Construction of pGBT9-VP3

For the construction of the bait plasmid, which enables the identification of apoptin-associating proteins by means of a yeast-two-hybrid system, plasmid pET-16b-VP3 (Noteborn, unpublished results) was treated with NdeI and BamHI. The 0.4 kb NdeI-BamHI DNA fragment was isolated from low-melting-point agarose.

Plasmid pGBT9 (Clontech Laboratories, Inc, Palo Alto, USA) was treated with the restriction enzymes EcoRI and BamHI. The about 5.4 kb DNA fragment was isolated and ligated with an EcoRI-NdeI linker and 0.4-kb NdeI-BamHI DNA fragment containing the apoptin-encoding sequences starting from its own ATG-initiation codon. The final construct containing a fusion gene of the GAL4-binding domain sequence and apoptin under the regulation of the yeast promoter ADH was called pGBT-VP3 and was proven to be correct by restriction-enzyme analysis and DNA-sequencing according to the Sanger method (1977).

All cloning steps were essentially carried out as described by Maniatis et al. (1992). The plasmid pGBT-VP3 was prurified by centrifugation in a CsCl gradient and column chromatography in Sephacryl S500 (Pharmacia).

### GAL4-activation domain-tagged cDNA library.

The expression vector pACT, containing the cDNAs from Epstein-Bar-virus-transformed human B cells fused to sequences for the GAL4 transcriptional activation domain, was used for detecting apoptin-associating proteins. The pACT c-DNA library is derived from the lambda-ACT cDNA library, as described by Durfee et al. 1993.

### Bacterial and Yeast strains

The E.coli strain JM109 was the transformation recipient for the plasmid pGBT9 and pGBT-VP3. The bacterial strain Electromax/DH10B was used for the transformation needed for the recovery the apoptin-associating pACT-cDNAs, and was obtained from GIBCO-BRL, USA.

The yeast strain Y190 was used for screening the cDNA library, and all transformations which are part of the used yeast-two-hybrid system.

### Media

For drug selections Luria Broth (LB) plates for E.coli were supplemented with ampicillin (50 microgram per ml). Yeast YPD and SC media were prepared as described by Rose et al. (1990).

### Transformation of competent yeast strain Y190 with plasmids pGBT-VP3 and pACT-cDNA and screening for beta-galactosidase activity.

The yeast strain Y190 was made competent and transformed according to the methods described by Klebe et al. (1983). The yeast cells were first transformed with pGBT-VP3 and subsequently transformed with pACT-cDNA, and these transformed yeast cells were grown on histidine-minus plates, also lacking leucine and tryptophan.

Hybond-N filters were layed on yeast colonies , which were histidine-positive and allowed to wet completely. The filters were lifted and submerged in liquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive was prepared by using the glusulase-alkaline lysis method as described by Hoffman and Winston (1987) and used to transform Electromax/DH10B bacteria via electroporation using a Bio-Rad GenePulser according the manufacturer's specifications.

Transformants were plated on LB media containing ampicillin.

### Isolation of apoptin-associating pACT clones

By means of colony-filter assay the colonies were lysed and hybridized to a radioactive-labeled 17-mer oligomer, which is specific for pACT (see also section Sequence analysis).

Plasmid DNA was isolated from the pACT-positive clones, and by means of XhoI digestion analysed for the presence of a cDNA insert.

### Sequence analysis

The subclones containing the sequences encoding apoptin-associating proteins were sequenced using dideoxy NTPs according to the Sanger method which was performed by Eurogentec, Nederland BV (Maastricht, The Netherlands). The used sequencing primer was a pACT-specific 17-mer comprising of the DNA-sequence 5'-TACCACTACAATGGATG-3'.

The sequences of the apoptin-associating proteins were compared with known gene sequences from the EMBL/Genbank.

### Results and discussion

Apoptin induces specifically apoptosis in transformed cells, such as cell lines derived from human tumors. To identify the essential compounds in this cell-transformation-specific and/or tumor-specific apoptosis pathway, a yeast genetic screen was carried out.

We have used a human cDNA library, which is based on the plasmid vector pACT containing the complete cDNA copies made from Epstein-Barr virus-transformed human B cells (Durfee et al., 1993).

### Construction of a bait plasmid expressing a fusion gene product of GAL4-DNA-binding domain and apoptin

To examine the existence apoptin-associating proteins by the human transformed/tumorigenic cDNA library, a so-called bait plasmid had to be constructed.

To that end, the complete apoptin-encoding region, flanked by about 40 basepairs downstream from the apoptin gene, was cloned in the multiple cloning site of plasmid pGBT9.

The final construct, called pGBT-VP3, was analysed by restriction-enzyme analysis and sequencing of the fusion area between apoptin and the GAL4-DNA-binding domain.

### A gene(fragment) encoding an apoptin-associating protein is determined by transactivation of a GAL4-responsive promoter in yeast

The apoptin gene is fused to the GAL4-DNA-binding domain of plasmid pGBT-VP3, whereas all cDNAs derived from the transformed human B cells are fused to the GAL4-activation domain of plasmid pACT. If one of the cDNAs will bind to apoptin, the GAL4-DNA-binding domain be in the vicinity of the GAL4-activation domain resulting in the activation of the GAL4-responsive promoter, which regulates the reporter genes HIS3 and LacZ.

The yeast clones containing plasmid expressing apoptin and a plasmid expressing an apoptin-associating protein(fragment) can grow on a histidine-minus medium and will stain blue in a beta-galactosidase assay. Subsequently, the plasmid with the cDNA insert encoding the apoptin-associating protein can be isolated and characterized.

Before we could do so, however, we have determined that transformation of yeast cells with pGBT-VP3 plasmid only or in combination with an empty pACT vector, did not result in the activation of the GAL4-responsive promoter.

### Identification of apoptin-associating proteins encoded by cDNAs derived from a human transformed B cell line

We have found yeast colonies, which upon transformation with pGBT-VP3 and pACT-CDNA were able to grow on a histidine-minus medium (also lacking leucine and tryptophan) and stained blue in a beta-galactosidase assay. These results indicate that these yeast colonies contain besides the bait plasmid pGBT-VP3 a pACT plasmid encoding for a potential apoptin-associating protein.

Plasmid DNA was isolated from these positive yeast colonies, which were transformed in bacteria. By means of an filter-hybridization assay using a pACT-specific labeled DNA-probe, the clones containing pACT plasmid could be determined. Subsequently, pACT DNA was isolated and digested with restriction enzyme XhoI, which is indicative for the presence of a cDNA insert. Finally, the pACT plasmids with a cDNA insert were sequenced.

### Description of apoptin-associating proteins

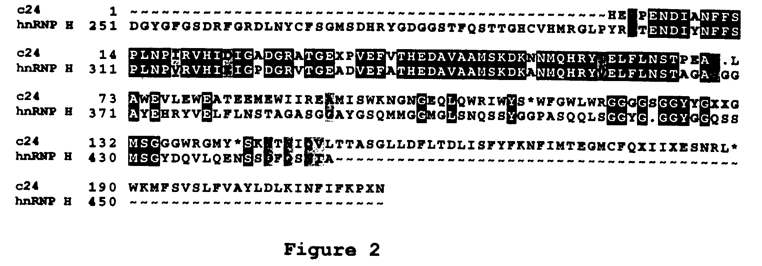

The yeast genetic screen for apoptin-associating proteins resulted in the detection of a human homolog of the hnRNP-H. The determined DNA sequence is shown in Fig. lThe amino acid sequence of the cloned hnRNP-H homolog is shown in Fig.2. Most likely, the cloned cDNA insert represents a new member of the family of (human) hnRNPs.

### Characteristics of hnRNP-H

The detected cDNA shows homology to part of hnRNP 1H, which is the abbreviation of heterogenous nuclear ribonucleoprotein H). hnRNPs bind to primary RNA transcripts (hnRNA or pre-mRNA), and are among the most abundant proteins in the nucleus. More than 20 hnRNPs have been discovered sofar, differing in size, localization, domains and nucleic acid binding specificity. Some hnRNPs were found to be confined to the nucleus, whereas others shuttle between the nucleus and cytoplasm (Dreyfuss et al., 1993).

Antibody staining shows a general nucleoplasmic localization, with little staining in nucleoli and electron-microscopy analysis localized hnRNPs, mainly to perichromatin fibrils. There is no evidence for free (not RNA-bound) hnRNPs in the nucleus. Many hnRNPs show preferential binding to certain RNA sequences, like stretches of identical bases or intron-splice sites. Almost all, including hnRNP-H have a common domain with which they can bind to RNA (Holzmann et al., 1997, Dreyfuss et al., 1993).

### The relationship between hnRNP-like proteins and apoptin.

The hnRNP-H protein interacts with the proteins CBP80 and CBP20, the components of the nuclear cap-binding complex. The hnRNP-H protein is closely related to hnRNP, which also binds to mentioned CBPs (Gamberi et al., 1997). Apoptin association will result in the inhibition of hnRNP activity. In this respect, it is interesting to mention that the CAV thereby developed a strategy, which makes the translation of the capsid protein cap-independent. Synthesis of VP2 and apoptin preceeds the production of the virus capsid protein. Most likely, apoptin will cause inhibition of capping activity by interference with hnRNP-H (Noteborn et al., 1991).

Upon various signals the expression of hnRNP-H can be up-regulated in transformed fibroblasts but not in normal cells (Honore et al., 1995). This seems to correlate with the apoptin activity in (human) transformed cells, whereas it does not in various normal human cells.

Interestingly, immunofluorescence-microscopy revealed that hnRNP are concentrated in discrete regions of the nucleoplasm, in contrast to the general nucleoplasmic distribution of previously characterized hnRNP (Matunis et al., 1994).

Co-localization studies in transformed human fibroblasts and keratinocytes with apoptin-specific and hnRNP-H monoclonal antibodies proved that apoptin is situated in a similar nuclear structure.

Thus far, hnRNP-H proteins were not linked with the apoptotic pathway. We provide evidence (apoptin as an example) that interference with the function of hnRNP-H results in the induction of apoptosis.

### Co-localization of VP2 and snRNPs.

Noteborn et al. (1997) have provided evidence that the other CAV-derived VP2 protein induces relatively weakly apoptosis in comparison to apoptin. Interestingly, however, VP2 has an enhancing effect on apoptin-induced apoptosis.

VP2 is like appoptin present in distinct structures in the nucleus. The structures of apoptin do not co-loxcalize with these of VP2. We have examined to which structures VP2 belong. Co-localization studies with an apoptin-specific and a snRNP-specific monoclonal antibody clearly revealed co-localization of snRNPs and VP2. In a parallel control experiment, it was proven that apoptin did not co-localize with snRNPs.

We have shown that the observed VP2 and apoptin activities, and their interactive behaviour, can be explained by the fact that both VP2 and apoptin interfere in the RNA processing pathway.

It is the first time that such a dualism of interference within the apoptotic pathway is linked to the apoptotic process.

### Conclusions

In conclusion, we have provided evidence that interference of specific factors with RNA processing, to be precise hnRNP-like proteins and/or snRNP-like proteins, will result in induction of apoptosis.

Therapies based on induction of apoptosis are possible if they succeed in the interference with the function of hnRNP-like and/or snRNP-like proteins. Examples of such interfering RNA-processing proteins are the CAV-derived proteins VP2 and apoptin.

### Other apoptin-associating proteins

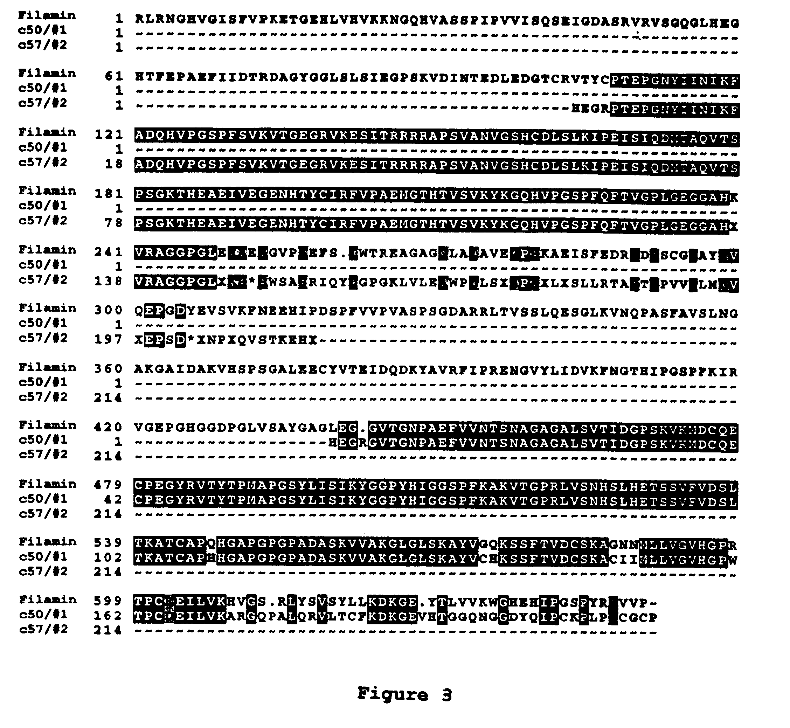

The genetic yeast screen with pGBT-VP3 as bait plasmid and pACT plasmid containing cDNAs from transformed human B cells also delivered the protein filamin. The protein filamin is localized within lamellipodia and filopodia. Filamin is one of the cross-linking proteins of actin. It may play an additional role of linking the cytoskeleton to cell-substratum adhesion sites (Matsudaira, 1994). Two independent filamin-like clones were found. The found associating amino acid sequence of the two filamin clones are shown in Figure 3.

### Description of the figures

Figure 1 shows the DNA sequence of the analysed region of the apoptin-associating clone hnRNP.

Figure 2 shows the amino acid sequence of the detected hnRNP-like protein, derived from clones No-1 and No-2. In addition, the three C-terminal amino acids (HEG) of the multiple cloning site of pACT are given to illustrate that the hnRNP-like amino acid sequence is in frame with the GAL4-activation domain. This feature proves that the hnRNP-like region is indeed synthesized in yeast cells.

Figure 3 shows the amino acids of the sequenced region of the apoptin-associating filamin clones. In addition, the three C-terminal amino acids (underlined) of the GAL4 activation domain are given to illustrate that the filamin-like amino acid sequence is in frame with the GAL4-activation domain. This feature proves that the filamin-like region is indeed synthesized.

### REFERENCES

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Danen-Van Oorschot, A.A.A.M., Fischer, D., Grimbergen, J.M., Klein, B., Zhuang, S.-M., Falkenburg, J.H.F., Backendorf, C., Quax, P.H.A., Van der Eb, J.A., and Noteborn, M.H.M. (1997). Proceedings National Academy Sciences, USA: 94, 5843-5847.
3. Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.
4. Dreyfuss, G., Matunis, M.J., Pinol-Roma, S., and Burd, C.G. (1993). HnRNP proteins and the biogenesis of mRNA. Annual Review Biochemistry 62, 289-321.
5. Duke, R.C., Ocjius, D.M., Young, J, D-E. (1996). Cell suicide in health and disease. Scientific American December 1996, 48-55.
6. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
7. Earnshaw, W.C., 1995. Nuclear changes in apoptosis. Current Opinion in Cell Biology 7, 337-343.
8. Fields, S. and Song, O.K. (1989). A novel genetic system to detect protein-protein interactions. Nature 340, 245-246.
9. Gamberi, C., Izaurralde, E., Beisel, C., and Mattaj, I.W. (1997). Interaction between the human nuclear cap-binding protein complex and hnRNP F.
10. Hockenberry, D.M. (1994). Bcl-2 in cancer, development and apoptosis. Journal of Cell Science, Supplement 18, 51-55.
11. Hoffman, C.S. and Winston, F. (1987). A ten-minute DNA preparation from yeast efficiently releases autonomous plasmids for transformation of Escherichia coili. Gene 57, 267-272.
12. Holzmann, K., Korosec, T., Gerner, C., Grimm, R., Sauermann, G. (1997). European Journal Biochemistry 244, 479-486.
13. Honore, B., Rasmussen, H.H., Vorum, H., Dejgaard, K., Liu, X., Gromov, P., Madsen, P., Gesser, B., Tommerup, N., and Celis, J.E. (1995). Journal of Biological Chemistry 270, 28780-28789.
14. Kerr, J.F.R., Winterford, C.M., and Harmon, B.V. (1994). Apoptosis: Its significance in cancer and cancer therapy. Cancer 73, 2013-2026.
15. Klebe, R.J., Harriss, J.V., Sharp, Z.D., and Douglas, M.G. (1983), A general method for polyethylene-glycol-induced genetic transformation of bacteria and yeast. Gene 25, 333-341.
16. Levine, A.J. (1997). p53, the cellular gatekeeper for growth and division. Cell 88, 323-331.
17. Maniatis, T., Fritsch, E.F., and Sambrook, J. (1982). Molecular Cloning: A Laboratory Manual. CSHL Press, New York, USA.
18. Matunis, M.J., Xing, J., Dreyfuss, G. (1994). The HNRNP-F protein: unique primary structure, nucleic acid-binding properties, and subcellular localization. Nucleic Acids Research 22, 1059-1067.
19. McDonell T.J., Meyn, R.E., Robertson, L.E. (1995). Implications of apoptotic cell death regulation in cancer therapy. Seminars in Cancer Biology 6, 53-60.
20. Noteborn, M.H.M. (1996). PCT application WO 96/41191. Apoptin induces apoptosis in human transformed and malignant cells but not in normal cells as essential characteristic for the development of an anti-tumor therapy.
21. Noteborn, M.H.M., and De Boer, G.F. (1996). Patent USA/no. 030, 335.
22. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
23. Noteborn, M.H.M., Hoeben, R.C., and Pietersen, A. (1997). A gene delivery vehicle expressing the apoptosis-inducing proteins VP2 and/or apoptin. European Patent Application no. 97201121.7
24. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
25. Noteborn, M.H.M., and Zhang, Y. (1997). Methods and means for determining the transforming capability of agents, for determining the predisposition of cells to become transformed and prophylactic treatment of cancer using apoptin-like activity. European Patent Application no. 97439
26. Paulovich, A.G., Toczyski, D., Hartwell, H. (1997). When checkpoints fail. Cell 88, 315-321.
27. Rose, M.D., Winston, F., and Hieter, P. (1990). Methods in yeast genetics. A laboratory course manual. Cold Spring Harbor Laboratory Press, Cold Spring Harbor, New York, USA.
28. Sachs, L. and Lotem, J. (1993). Control of programmed cell death in normal and leukemia cells: New implications for therapy. Blood 82, 15-21.
29. Sanger, F., Nicklen, S., and Coulsen, A.R. (1977). DNA sequencing with chain-terminating inhibitors. Proceedings National Academic Sciences USA 74, 5463-5467.
30. Steller, H. (1995). Mechanisms and genes of cellular suicide. Science 267, 1445-1449.
31. Telford, W.G., King, L.E., Fraker, P.J. (1992). Comparative evaluation of several DNA binding dyes in the detection of apoptosis-associated chromatin degradation by flow cytometry. Cytometry 13, 137-143.
32. Teodoro, J.G. and Branton, P.E. (1997). Regulation of apoptosis by viral gene products. Journal of Virology 71, 1739-1746.
33. Thompson, C.B. (1995). Apoptosis in the pathogenesis and treatment of disease. Science 267, 1456-1462.
34. White, E. (1996). Life, death, and the pursuit of apoptosis. Genes and development 10, 1-15.
35. Wyllie, A.H. (1995). The genetic regulation of apoptosis. Current Opinion in Genetics and Development 5, 97-104.
36. Wyllie, A.H., Kerr, J.F.R., Currie, A.R. (1980). Cell death: The significance of apoptosis. International Review of Cytology 68, 251-306.
37. Yang, X., Hubbard, E.J.A., and Carlson, M. (1992). A protein kinase substrate identified by the two-hybrid system. Science 257, 680-682.
38. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.
39. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. A recombinant and/or isolated nucleic acid molecule encoding a member of the family of hnRNP proteins involved in RNA processing comprising at least a functional part of the sequence of figure 1 or a sequence having at least 60, preferably 70, preferably 80, more preferably 90% homology with said sequence.

2. An expression vector comprising a nucleic acid according to claim 1.

3. A vector according to claim 2 further comprising a nucleic acid sequence encoding apoptin-like activity.

4. A vector according to claim 3 wherein said apoptin-like activity is apoptin or a functional fragment and/or derivative thereof.

5. A recombinant and/or isolated nucleic acid molecule encoding an antisense recombinant molecule which can hybridize with a recombinant acid molecule according to claim 1.

6. An expression vector comprising an isolated nucleic acid molecule encoding an antisense recombinant molecule according to claim 5.

7. An expression vector according to claim 6 further comprising a sequence encoding an antisense molecule for a nucleic acid encoding a component of an snRNP.

8. A gene delivery vehicle comprising an expression vector according to any one of claims 2-4, 6 or 7.

9. Method for identifying apoptotic agents comprising the use of nucleic acid molecules encoding members of the hnRNP-like family.

10. Apoptotic agent obtainable by a method according to claim 9.

11. A method for inducing apoptosis in a cell comprising providing said cell with antagonistic activity for hnRNP components

12. A method for inducing apoptosis in a cell comprising providing said cell with antagonistic activity for snRNP components

13. A method according to claim 11 further comprising said cell with antagonsistic activity for snRNP components.

14. A method according to claim 11 or 13 wherein the hnRNP antagonistic activity is apoptin-loike activity.

15. A method according to claim 12 or 13 wherein said snRNP antagonistic activity is VP2-like activity.

16. A gene delivery vehicle encoding VP2-like and apoptin-like activity.

17. A proteinaceous substance comprising at least a functional and/or specific part of the sequence of figure 2 or a functional equivalent thereof.
